# EUROPEAN PATENT SPECIFICATION

(11) **EP 3 704 707 B1**
(45) Date of publication and mention of the grant of the patent: **10.01.2024**
(21) Application number: 18800551.6
(22) Date of filing: 02.11.2018
(51) Int. Cl.: G16H 30/40, G16H 50/70

(54) **A METHOD AND APPARATUS FOR ANALYSING ECHOCARDIOGRAMS**
VERFAHREN UND VORRICHTUNG ZUR UNTERSUCHUNG VON ECHOKARDIOGRAMMEN
PROCÉDÉ ET APPAREIL D'ANALYSE D'ÉCHOCARDIOGRAMMES

(30) Priority: 02.11.2017 US 201762580491 P
(43) Date of publication of application: 09.09.2020
(73) Proprietor: Koninklijke Philips N.V., 5656 AG Eindhoven (NL)
(72) Inventor: SEVENSTER, Merlijn, 5656 AE Eindhoven (NL); SITEK, Arkadiusz, 5656 AE Eindhoven (NL)
(74) Representative: Philips Intellectual Property & Standards
(86) International application number: PCT/EP2018/079963
(87) International publication number: WO 2019/086586

(56) References cited:
- WO-A1-2017/056078
- WEILIN HUANG ET AL: "Temporal HeartNet: Towards Human-Level Automatic Analysis of Fetal Cardiac Screening Video", ARXIV.ORG, CORNELL UNIVERSITY LIBRARY, 201 OLIN LIBRARY CORNELL UNIVERSITY ITHACA, NY 14853, 3 July 2017 (2017-07-03), XP080774039, DOI: 10.1007/978-3-319-66185-8_39

## Description

### Technical Field of the Invention

The invention relates to the field of echocardiogram analysis and in particular, to a method and apparatus for analysing echocardiograms and training a predictive model to determine a class for a pair of consecutive echocardiograms.

### Background to the Invention

Echocardiogram examinations ("echo") are a type of common non-invasive imaging technique that is the primary modality for viewing and monitoring a patient's cardiac function. To perform an echocardiogram examination, a medical technician holds a transducer on the patient's chest. The transducer generates high frequency sound waves which are reflected back to the transducer by the different structures of the heart. This reflection of the waves, i.e. echoes, is used to form an image. One echo comprises one or more sequences, each interrogating the heart anatomy from a different angle and at a different zoom level.

Echocardiogram can be used to measure the size and/or the structure of the heart, for example the thickness of heart muscles and how blood flows through the heart chambers (when combined with a Doppler technique). Through echocardiogram examinations, tumours or emboli in the heart can be identified. In addition, structural abnormalities of the heart wall, the valves, and the blood vessels can be visualised using echocardiograms. This technique is therefore useful for diagnosing congenital heart diseases (e.g. ventricular septal defect), cardiomyopathies, and aneurysms.

Although an echocardiogram provides a significant amount of information to the physician relating to the patient's heart condition, it may be challenging for a physician to examine the vast amount of information and formulate a diagnosis on this basis, especially when there is a large number of patients.

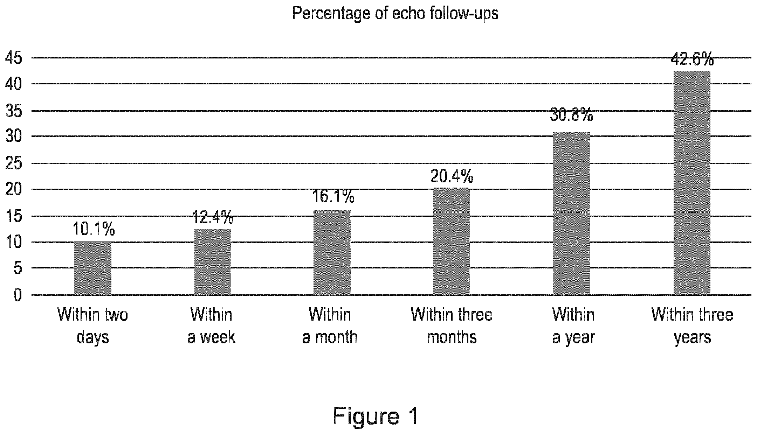

Echocardiogram examinations are costly, as they require acquisitions by sonographers and interpretation by subspecialised cardiologists. The volume of echocardiogram examinations performed annually continues to grow. Currently, a significant portion of patients would undergo a follow-up echocardiogram examination so that the physician (or other medical personnel) can observe for any changes in the image from the initial examination. For example, at the University of Chicago Medical Centre, more than 10% of the patients have a follow-up echocardiogram examination within one week and around 40% of the patients have a follow-up echocardiogram examination within three years. These and other follow-up statistics are presented in **Figure 1****.**

Although there are continued efforts to improve image processing techniques to improve and assist the interpretation of echocardiograms by cardiologists, such as improvements to the capability of creating specific measurements and assessments, there are still challenges in developing automated interpretation of echocardiogram data. One of the underlying reasons is that the human heart can be pathological in a number of different ways. The set of differential diagnoses therefore spans a vast search space, which is potentially too large for automated means.

### Summary of the Invention

As noted above, there are difficulties in developing automated interpretation of echocardiograms due to a number of constraints. One of the ways to simplify this interpretation is to perform automated comparison of consecutive echocardiograms instead of performing interpretation of a single echocardiogram, since the comparison relies on binary comparison (e.g. identifying any significant changes compared to the most recent prior echocardiogram). However, the automated comparison of echocardiograms is difficult in general because the three-dimensional views may correspond to different planes through the heart. In other words, the prior and current echocardiograms may correspond to different (shifted) planes and therefore show different visible structures of the heart.

Significant progress has been made recently on the application of artificial convolutional neural networks (e.g. Convolutional Deep Learning, CDL) to image recognition/classification. In particular, it has been shown that neural networks can be applied to the 'raw' pixel data in images without the need for 'feature-engineering', i.e. the process of converting lower-level information into higher-level information about the underlying image data. The rise in popularity of CDL contributed to increase interest in the general machine learning method in applications in various domains, in particular in echocardiogram analysis.

It would be advantageous to perform automated comparison of consecutive echocardiograms so as to identify whether there is a change between the consecutive echocardiograms. It would also be desirable to perform echocardiogram analysis based on a predictive model that is trained using artificial convolutional neural network(s) so as to achieve a more accurate analysis. Document WO 2017/056078 A1 is considered as closest prior art. Said document discloses the comparison of consecutive echocardiograms based on finding data inputted by a user, however fails to disclose to automatically determine whether a change occurred by comparing the current echocardiogram image data to prior echocardiogram image data of a patient.

To better address one or more of these concerns, in a first aspect there is provided a computer-implemented method for analysing echocardiograms, the method comprising: obtaining a plurality of pairs of consecutive echocardiograms for a plurality of subjects from a database, each echocardiogram having an associated indication, comprising one or more observational or diagnostic statements input by a user of the content of the echocardiogram;analysing each pair of consecutive echocardiograms to determine an associated class by automatic comparison between said observational or diagnostic statements associated with each of the pair of consecutive echocardiograms, the class indicating whether there is a change or no change between the consecutive echocardiograms in the pair, said change between the consecutive echocardiograms refers to a change in the cardiac function and/or structure of the heart as represented in the consecutive echocardiograms;for each pair of consecutive echocardiograms, determining an abstract representation of each echocardiogram by performing at layers of a convolutional deep learning network one or more convolutions and/or reductions on the echocardiograms in the pair, the abstract representation comprising one or more features indicative of the class of the pair; andtraining a predictive model to determine the class for a new pair of echocardiograms based on the abstract representations for the plurality of pairs of consecutive echocardiograms.

In some embodiments, the method may further comprise temporally aligning the pair of consecutive echocardiograms with respect to a cardiac cycle.

In some embodiments, each echocardiogram may comprise a plurality of image frames, the method further comprising interpolating one or more image frames for one or both of the echocardiograms such that each echocardiogram comprises the same number of image frames.

In the present invention, the content of the echocardiogram comprises one or more observational or diagnostic statements input by a user.

In some embodiments, the method may further comprise: receiving a new echocardiogram for a subject; obtaining a previous echocardiogram for the subject; using the predictive model to determine a class for the new echocardiogram and the previous echocardiogram. In some of these embodiments, the method may further comprise controlling an interface to provide a notification to a user if the determined class of the new pair of echocardiograms indicates there is a change. Moreover, in some of these embodiments, the step of obtaining a previous echocardiogram may comprise obtaining an associated indication of the content of the previous echocardiogram, the method further comprising controlling an interface to provide the associated indication and/or the content to a user if the determined class of the new pair of echocardiograms indicates there is no change.

In some embodiments, each echocardiogram may comprise a plurality of sequences, each sequence representing a different view of the subject, and wherein analysing each pair of consecutive echocardiograms may further comprise joining the plurality of sequences of each echocardiogram to form a single image.

In some embodiments, each echocardiogram may comprise a plurality of sequences, each sequence representing a different view of the subject, and wherein analysing each pair of consecutive echocardiograms may further comprise comparing each of the plurality of sequences in one echocardiogram of the pair with each of the plurality of sequences in the other echocardiogram of the pair.

In some embodiments, each echocardiogram may comprise a plurality of sequences, each sequence representing a different view of the subject, and wherein the method may further comprise associating a view tag with each of the plurality of sequences, wherein the view tag may be indicative of the view of the subject represented by the sequence. A view tag may be one of parasternal long axis, parasternal short axis, apical four chamber, apical five chamber, apical two chamber, apical three chamber, sub costal, and supra sternal notch.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

In a third aspect there is provided an apparatus for analysing echocardiograms, the apparatus comprising a processor configured to:obtain a plurality of pairs of consecutive echocardiograms for a plurality of subjects from a database, each echocardiogram having an associated indication, comprising one or more observational or diagnostic statements input by a user of the content of the echocardiogram;analyse each pair of consecutive echocardiograms to determine an associated class by automatic comparison between said observational or diagnostic statements associated with each of the pair of consecutive echocardiograms, the class indicating whether there is a change or no change between the consecutive echocardiograms in the pair, said change between the consecutive echocardiograms refers to a change in the cardiac function and/or structure of the heart as represented in the consecutive echocardiograms;for each pair of consecutive echocardiograms, determine an abstract representation of each echocardiogram by performing at layers of a convolutional deep learning network one or more convolutions and/or reductions on the echocardiograms in the pair, the abstract representation comprising one or more features indicative of the class of the pair; andtrain a predictive model to determine the class for a new pair of echocardiograms based on the abstract representations for the plurality of pairs of consecutive echocardiograms.

In some embodiments, the processor is further configured to temporally aligning the pair of consecutive echocardiograms with respect to a cardiac cycle.

In some embodiments, each echocardiogram may comprise a plurality of image frames, the processor being further configured to interpolate one or more image frames for one or both of the echocardiograms such that each echocardiogram comprises the same number of image frames.

In the present invention, the content of the echocardiogram comprises one or more observational or diagnostic statements input by a user.

In some embodiments, the processor is further configured to: receive a new echocardiogram for a subject; obtain a previous echocardiogram for the subject; use the predictive model to determine a class for the new echocardiogram and the previous echocardiogram. In some of these embodiments, the processor is further configured to control an interface to provide a notification to a user if the determined class of the new pair of echocardiograms indicates there is a change. Moreover, in some of these embodiments, the processor is further configured to obtain an associated indication of the content of the previous echocardiogram, the processor being further configured to control an interface to provide the associated indication and/or the content to a user if the determined class of the new pair of echocardiograms indicates there is no change.

In some embodiments, each echocardiogram may comprise a plurality of sequences, each sequence representing a different view of the subject, and wherein the processor is configured to analyse each pair of consecutive echocardiograms by joining the plurality of sequences of each echocardiogram to form a single image.

In some embodiments, each echocardiogram may comprise a plurality of sequences, each sequence representing a different view of the subject, and wherein the processor is configured to analyse each pair of consecutive echocardiograms may further comprise comparing each of the plurality of sequences in one echocardiogram of the pair with each of the plurality of sequences in the other echocardiogram of the pair.

In some embodiments, each echocardiogram may comprise a plurality of sequences, each sequence representing a different view of the subject, and wherein the processor is further configured to associate a view tag with each of the plurality of sequences, wherein the view tag is indicative of the view of the subject represented by the sequence. A view tag may be one of parasternal long axis, parasternal short axis, apical four chamber, apical five chamber, apical two chamber, apical three chamber, sub costal, and supra sternal notch.

These and other aspects will be apparent from and elucidated with reference to the embodiment(s) described hereinafter. The invention is defined by the appended claims.

### Brief Description of the Drawings

For a better understanding of the embodiments described herein, and to show more clearly how they may be carried into effect, reference will now be made, by way of example only, to the accompanying drawings, in which:
Figure 1 is a graph showing cumulative percentages of patients who had follow-up echocardiogram examinations at the University of Chicago Medical Centre;
Figure 2 is a block diagram of an apparatus for analysing echocardiograms according to an embodiment;
Figure 3 illustrates a method for analysing echocardiograms according to an embodiment; and
Figure 4 is a schematic diagram of a convolutional deep network for analysing echocardiograms according to an example embodiment.

### Detailed Description of the Embodiments

As noted above, there is provided an improved method and apparatus for analysing echocardiograms which overcomes existing problems.

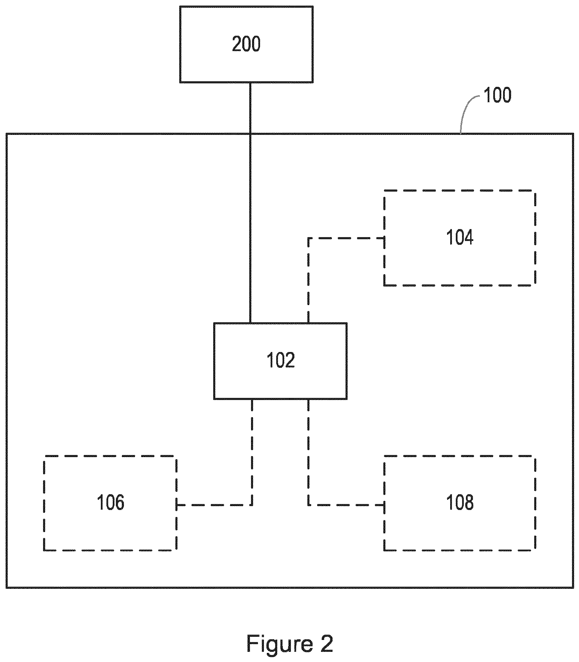

**Figure 2** shows a block diagram of an apparatus 100 according to an embodiment for analysing echocardiograms. In some embodiments, the apparatus may be a computer, a server, or a laptop etc.

As illustrated in Figure 2, the apparatus 100 comprises a processor 102 that controls the operation of the apparatus 100 and that can implement the method described herein. The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method according to embodiments described herein.

Briefly, the processor 102 is configured to obtain a plurality of pairs of consecutive echocardiograms for a plurality of subjects from a database 200, each echocardiogram having an associated indication of the content of the echocardiogram. The processor 102 is then configured to analyse each pair of consecutive echocardiograms to determine an associated class, the class indicating whether there is a change or no change between the consecutive echocardiograms in the pair. For each pair of the consecutive echocardiograms, the processor 102 is configured to determine an abstract representation of each echocardiogram by performing one or more convolutions and/or reductions on the echocardiograms in the pair. The processor 102 is further configured to train a predictive model to determine a class for a new pair of echocardiograms based on the abstract representations for the plurality of pairs of consecutive echocardiograms.

The processor 102 can be implemented in numerous ways, with software and/or hardware, to perform the various functions described below. The processor 102 may comprise one or more microprocessors or digital signal processor (DSPs) that may be programmed using software or computer program code to perform the required functions and/or to control components of the processor 102 to effect the required functions. The processor 102 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In some embodiments, the apparatus may further comprise at least one user interface 104. Alternatively or in addition, at least one user interface 104 may be any user interface that enables a user of the apparatus 100 to provide user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may also comprise a memory 106 configured to store program code that can be executed by the processor 102 to perform the method described herein. The memory 106 may comprise cache or system memory including volatile and non-volatile computer memory such as random access memory (RAM) static RAM (SRAM), dynamic RAM (DRAM), read-only memory (ROM), programmable ROM (PROM), erasable PROM (EPROM), and electrically erasable PROM (EEPROM).

Alternative or in addition, one or more memories 106 may be external to (i.e. separate to or remote from) the apparatus 100. For example, one or more memories 106 may be part of another device. A memory 106 can be used to store images (including echocardiograms), information, data, signals and measurements acquired or made by the processor 102 of the apparatus 100 or from any interfaces, memories or devices that are external to the apparatus 100. For example, a memory 106 may be used to store (for example, in a local file) one or more prior or current echocardiograms for use in the analysis. The processor 102 may be configured to control a memory 106 to store the one or more prior or current echocardiograms for use in the analysis.

In some embodiments, the database 200 of the plurality of pairs of consecutive echocardiograms for a plurality of subjects may be stored in the memory 106, but in other embodiments the database 200 can be separate from the apparatus 100.

In some embodiments, the apparatus 100 may also comprise a communications interface (circuitry) 108 for enabling the apparatus 100 to communicate with any interfaces, memories and devices that are internal or external to the apparatus 100. The communications interface 108 may communicate with any interfaces, memories and devices that are internal or external to the apparatus 100. The communications interface 108 may communicate with any interfaces, memories and devices wirelessly or via a wired connection. For example, in an embodiment where one or more user interfaces 104 are external to the apparatus 100, the communications interface 108 may communicate with the one or more external user interfaces 104 wirelessly or via a wired connection. Similarly, in an embodiment where one or more memories 106 are external to the apparatus 100, the communications interface 108 may communicate with the one or more external memories 106 wirelessly or via a wired connection.

It will be appreciated that Figure 2 only shows the components required to illustrate this aspect of the invention, and in a practical implementation the apparatus 100 may comprise additional components to those shown. For example, the apparatus 100 may comprise a battery or other power supply for powering the apparatus 100 or means for connection the apparatus 100 to a mains power supply.

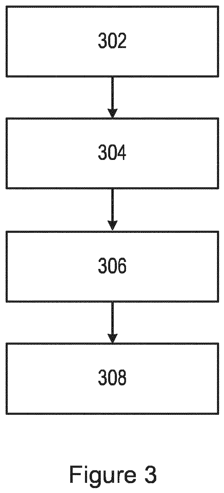

**Figure 3** shows a computer-implemented method 300 for analysing echocardiograms according to an embodiment. The illustrated method 300 can generally be performed by or under the control of the processor 102 of the apparatus 100.

With reference to Figure 3, at block 302, a plurality of pairs of consecutive echocardiograms for a plurality of subjects are obtained from the database 200. The processor 102 can obtain the plurality of pairs by retrieving them from the database 200. Each echocardiogram in the plurality of pairs of consecutive echocardiograms has an associated indication of the content of the echocardiogram. In some embodiments, the content of the echocardiogram may comprise one or more observational or diagnostic statements input by the user. In more detail, these statements can be separated into two different categories: discrete statements (e.g. "left ventricle is normal") and measurement statements (e.g. "left ventricular ejection fraction is 45.0%"). In some embodiments, an internal identifier may be associated with each of the one or more observational or diagnostic statements.

In some embodiments, the processor 102 of the apparatus 100 may be configured so as to detect a change based on the observational or diagnostic statements of a pair of consecutive echocardiograms. For example, the processor 102 may be configured to detect that there is a change between the statement "left ventricular ejection fraction is 45.0%" associated with an echocardiogram in a pair and the statement "left ventricular ejection fraction is 60.0%" associated with the other echocardiogram in the pair. Therefore, in some of these embodiments, prior to block 304, the method may further comprise triggering analysis of a pair of echocardiograms when a change between the statements associated with each of the echocardiograms in the pair is detected. In more detail, in some of these embodiments, a predetermined difference threshold may be employed such that small changes (lower than the predetermined difference threshold) may be neglected. For example, a predetermined difference threshold of "less than 10.0% in ejection fraction" may be set, such that if an echocardiogram in a pair of consecutive echocardiograms comprises an associated indication statement that "left ventricular ejection fraction is 45.0%" and the other echocardiogram in the same pair of consecutive echocardiograms comprises an associated indication statement that "left ventricular ejection fraction is 50.0%", the processor 102 may be configured to neglect this difference (i.e. 5.0%, which is less than 10.0%) between statements.

Moreover, in some of these embodiments where the processor 102 is configured to detect a change based on the observational or diagnostic statements of a pair of echocardiograms, prior to block 304, the method may further comprise triggering analysis of a pair of echocardiograms when it is determined that there is no change between the statements associated with each of the echocardiograms. As such, the analysis of the pair of echocardiograms can provide a further confirmation that there is no change between the pair of echocardiograms.

In some embodiments, each echocardiogram may comprise a plurality of image frames. Specifically, for each echocardiogram, the plurality of image frames may each correspond to a temporal instance of the heart during a cardiac cycle. For example, a portion of the image frames may correspond to systole part of the cardiac cycle and another portion of the image frames may correspond to diastole part of the cardiac cycle.

In these embodiments, the method can further comprise temporally aligning the pair of consecutive echocardiograms with respect to a cardiac cycle. By performing this temporal alignment of the consecutive echocardiograms, the accuracy of the analysis of the echocardiograms can be improved since the image frames in the pair of echocardiograms are synchronised such that the part of the cardiac cycle in the image frames in one of the pair of echocardiograms temporally correspond to the part of the cardiac cycle in the image frames in the other echocardiogram in the pair. Furthermore, in these embodiments, the method may further comprise interpolating one or more image frames for one or both of the echocardiograms such that each echocardiogram comprises the same number of image frames.

At block 304 of Figure 3, each pair of consecutive echocardiograms are analysed in order to determine an associated class. The determination at block 304 can be performed by automatic comparison between the observational or diagnostic statements associated with each of the pair of consecutive echocardiograms, or performed manually (i.e. a user observing the pair of echocardiograms and indicating, by providing a label, whether there is an observed change between the pair of echocardiograms). The class indicates whether there is a change or no change between the consecutive echocardiograms in the pair. A change between the consecutive echocardiograms refers to a change in cardiac function and/or structure of the heart as represented in the consecutive echocardiograms, rather than a change in visual representation (e.g. noise level, texture, view positioning) between the consecutive echocardiograms. The determined associated class of each pair of consecutive echocardiograms and the respective pair of consecutive echocardiograms form a ground truth. The ground truth associated with each pair of consecutive echocardiograms is indicative of whether there is a change in cardiac function and/or structure of the heart between the pair of consecutive echocardiograms and may form a basis of the predictive model that is trained at block 308.

In some embodiments, each echocardiogram may comprise a plurality of sequences (i.e. a plurality of sequences of images), each sequence representing a different view of the subject. In these embodiments, analysing each pair of consecutive echocardiograms may comprise joining the plurality of sequences of each echocardiogram (e.g. side by side) so as to form a single image. In other words, the plurality of sequences of each echocardiogram is represented by a single image which consists of images of the plurality of sequences. This single image can then be analysed in the same manner as an echocardiogram with a single view. Alternatively, in these embodiments, analysing each pair of consecutive echocardiograms may comprise performing convolution operations on the plurality of sequences individually and mixing information at a fully connected layer in a convolutional deep learning network.

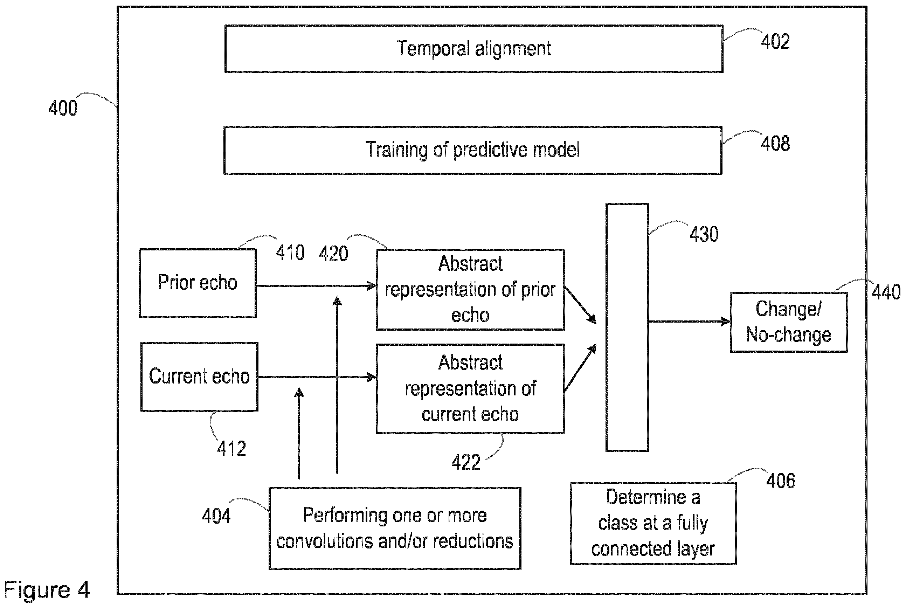

Also alternatively, in some embodiments where each echocardiogram comprises a plurality of sequences, each sequence representing a different view of the subject, a pairwise comparison may be used so as to determine an associated class. In more detail, if one of the echocardiograms in the pair comprises sequences S₁, S₂,..., Sₘ, and the other of the echocardiogram comprises sequences T₁, T₂,...Tₙ, the analysis of the pair of consecutive echocardiograms may comprise first comparing S₁ with each of T₁, T₂,...Tₙ, and then comparing S₂ with each of T₁, T₂,...Tₙ, and so forth such that each sequence Sᵢ in a first echocardiogram among the pair of echocardiograms is compared with each sequence Tⱼ in a second echocardiogram among the pair of echocardiograms. In some embodiments where the method is implemented in a convolutional deep learning network (such as one illustrated in Figure 4), each of the sequences S₁, S₂,..., Sₘ and T₁, T₂,...Tₙ may be associated with each of a plurality of input nodes of the convolutional deep learning network.

In embodiments where each echocardiogram comprises a plurality of sequences and each sequence represents a different view of the subject, the method may further comprise associating a view tag with each of the plurality of sequences, wherein the view tag is indicative of the view of the subject represented by the sequence. In detail, each of the plurality sequences in the echocardiogram may be analysed using current image processing technique to determine a view of the subject, such that respective sequences can be associated with one of the view tags: parasternal long axis (PLAX), parasternal short axis (PSAX), apical four chamber (AP4), apical five chamber (AP5), apical two chamber (AP2), apical three chamber (AP3), sub costal (SC), and supra sternal notch (SSN). Furthermore, in these embodiments where each of the plurality of sequences is associated with a respective view tag, the method may further include analysing a pair of consecutive echocardiograms (amongst the plurality of pairs of consecutive echocardiograms) only if the view tags of the pair of consecutive echocardiograms are the same, e.g. both of the view tags of the pair of echocardiograms is AP2. By only comparing sequences with the matching/same view tags, efficiency of the method can be improved.

At block 306 of Figure 3, for each pair of consecutive echocardiograms, an abstract representation of each echocardiogram is determined by performing one or more convolutions and/or reductions on the echocardiograms in the pair. A convolution (or filtering) operation is performed based on an image processing algorithm which allows a convolved value of each pixel in an image to be computed. To compute a convolved value of a pixel, a linear combination of all neighbouring pixels is computed with linear coefficients defined by a plurality of parameters of a convolutional kernel. The parameters of the convolutional kernels may be determined in the network training process of a predictive model. A reduction operation reduces the number of pixels in an image by enlarging the pixels and obtaining an average value of the pixels or a maximum value of the pixels. Convolutions and/or reductions may be performed at layers of a convolutional deep learning network and the number of layers corresponding to convolutions and/or reductions in a convolutional deep learning network may vary. After convolutions and/or reductions are performed, the original images(s) may be transformed and may be represented by a set of features that are characterised by a plurality of numbers. This set of features may be further associated with a decision making node in the convolutional deep learning network so as to produce an outcome decision.

In some embodiments, the determination of an abstract representation is performed separately for each of the echocardiograms in the pair. The abstract representation comprises one or more features indicative of the class of the pair. For example, the abstract representation may comprise a vector which corresponds to a feature vector generated by a predictive model, the feature vector being indicative of a change in structural and/or functional properties of the heart of a subject. As another example, the abstract representation may comprise data which corresponds to a set of data generated through a predictive model, the set of data being indicative of a change in structural and/or functional properties of the heart of a subject. This predictive model may be the same predictive model that is trained in block 308.

At block 308 of the Figure 3, a predictive model is trained based on the abstract representations for the plurality of pairs of consecutive echocardiograms, so as to determine a class for a new pair of echocardiograms. In some embodiments, the predictive model may be a convolutional deep learning model. The trained predictive model may also be used to determine a class for a new pair of echocardiograms based on study of previous pairs of echocardiograms of similar subjects if the time interval between the new pair of echocardiograms exceeds a predetermined time threshold (e.g. 7 days).

Although not illustrated in Figure 3, the method according to some embodiments may further comprise receiving a new echocardiogram for a subject, obtaining a previous echocardiogram for the subject, and using the predictive model to determine a class for the new echocardiogram and the previous echocardiogram. In some cases the previous echocardiogram and the new echocardiogram may be respectively referred to as the "prior echocardiogram" and the "current echocardiogram". In these embodiments the method may also further comprise controlling an interface to provide a notification to a user if the determined class of the new pair of echocardiograms indicates there is a change. Also, in these embodiments, the method may also further comprise highlighting the pair of echocardiograms on an interface if the determined class of the new pair of echocardiograms indicates there is a change. The previous echocardiogram may be obtained by retrieving it from the memory 106, or by retrieving it from database 200. The new echocardiogram may be received directly from a device that creates echocardiograms of a subject, or another device that stores the new echocardiogram prior to processing by the apparatus 100. Alternatively, the new echocardiogram can be received by the processor 102 from the memory 106.

Moreover, in these embodiments where the new echocardiogram is received and the previous echocardiogram of the subject is obtained, the step of obtaining the previous echocardiogram comprises obtaining an associated indication of the content of the previous echocardiogram. In these embodiments, the method may further comprise controlling an interface to provide the associated indication of the content of the previous echocardiogram to a user if the determined class of the new pair of echocardiograms indicates there is no change. Therefore, if it is determined that there is no change between the previous echocardiogram and the new echocardiogram, the associated indication of the content of the previous echocardiogram is made available to be applied also to the new echocardiogram (as there is no change) and to be viewed by a user (e.g. a medical technician) easily without the need to perform detailed analysis or interpretation of the new echocardiogram.

In some embodiments, the method as illustrated in Figure 3 may be applied so as to observe specific anatomy changes, rather than to observe changes of the heart as a whole. For example, in some embodiments, the method may be applied to determine a class that indicates whether there is a change or no change in a particular part of the heart, e.g. the left ventricle, between the consecutive echocardiograms in the pair. In addition, in some embodiments, the method as illustrated in Figure 3 may be applied so as to observe structural and/or functional changes of other organs of a subject, such as liver, gallbladder, abdomen, rectum, etc. The same principles can be applied to fetal ultrasound.

**Figure 4** is a schematic diagram of a convolutional deep learning network 400 for analysing echocardiograms according to an example embodiment. The convolutional deep learning network 400 illustrated in Figure 4 is a more detailed implementation of the apparatus 100 described earlier with reference to Figure 2. In the example embodiment illustrated in Figure 4, the processor 102 of the apparatus 100 is configured to perform blocks 402, 404, and 406.

As illustrated in Figure 4, a pair of consecutive echocardiograms, i.e. a prior echocardiogram 410 and a current echocardiogram 412, is retrieved from a database (e.g. database 200) of the system 400. Similar to the pair of consecutive echocardiograms described above in relation to Figure 3, each of the prior echocardiogram 410 and the current echocardiogram 412 has an associated indication of the content of the echocardiogram. The content of the echocardiogram may comprise one or more observational or diagnostic statements input by a user, such as "left ventricle is normal" or "left ventricle is severely reduced". Moreover, in this embodiment each of the prior echocardiogram 410 and the current echocardiogram 412 comprises a plurality of image frames, each corresponding to a temporal instance of the heart during a cardiac cycle.

As mentioned earlier, in the example embodiments illustrated in Figure 4, the processor 102 is configured to perform blocks 402, 404, and 406. Briefly, the processor 102 is configured to perform temporal alignment of the pair of consecutive echocardiograms 410, 412 with respect to a cardiac cycle, perform one or more convolutions and/or reductions to determine an abstract representation of each of the prior echocardiogram 410 and the current echocardiogram 412, and determine an associated class of the pair of consecutive echocardiograms 410, 412 at a fully connected layer of the convolutional deep learning network 400. The details of each of these blocks 402, 404 and 406 are explained in further detail below.

At block 402 of Figure 4, the processor 102 is configured to temporally align the pair of consecutive echocardiograms 410, 412 with respect to a cardiac cycle. Therefore, the content of image frames in the prior echocardiogram 410 will temporally correspond to the content of image frames in the current echocardiogram 412 and they can be compared in a more straight-forward manner in subsequent processes, e.g. at block 406 where an associated class of the pair of echocardiograms is determined.

At block 404 of Figure 4, the processor 102 is configured to determine an abstract representation of the each of the prior echocardiogram 410 and the current echocardiogram 412 by performing one or more convolutions and/or reductions on the echocardiogram. The abstract representations 420, 422 for the prior echocardiogram and the current echocardiogram each comprise one or more features indicative of the class of the pair of echocardiograms.

At block 406 of Figure 4, the processor 102 is configured to determine an associated class of the pair of consecutive echocardiograms 410, 412 at a fully connected layer of the convolutional deep learning network 400, where image subtraction or any other operation that integrates information from the abstract representations 420, 422 in a way that is optimal for determining the associated class of the pair of consecutive echocardiograms. This fully connected layer is represented as block 430 in Figure 4 and allows information from the abstract representations 420, 422, which are originally results of separate processes, to be integrated. The outcome of the determination, i.e. the associated class of the pair of consecutive echocardiograms 410, 412 which indicates whether there is a change or no change between the consecutive echocardiograms 410, 412, is represented by block 440 ("change/no-change").

Furthermore, in the convolutional deep learning network 400 according to the present embodiment, the processor 102 is also configured to train a predictive model at block 408 to determine a class for a new pair of echocardiograms, based on the abstract representations 420, 422 for the pair of consecutive echocardiograms 410, 412. In more detail, the accuracy of the determination of an associated class for a new pair of echocardiograms can be improved by optimising the operations for mixing information based on the abstract representations 420, 422.

There is therefore provided an improved method and apparatus for analysing echocardiograms. The method and apparatus according to embodiments described herein automatically compares consecutive echocardiograms based on a database comprising annotated echocardiograms (i.e. echocardiograms that have associated indications of their content) and detecting relevant changes while ignoring inconsequential changes (e.g. due to different noise levels or view positioning).

Variations to the disclose embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1. A computer-implemented method for analysing echocardiograms, the method comprising:
obtaining (302) a plurality of pairs of consecutive echocardiograms for a plurality of subjects from a database (200), each echocardiogram having an associated indication, comprising one or more observational or diagnostic statements input by a user of the content of the echocardiogram;
analysing (304) each pair of consecutive echocardiograms to determine an associated class by automatic comparison between said observational or diagnostic statements associated with each of the pair of consecutive echocardiograms, the class indicating whether there is a change or no change between the consecutive echocardiograms in the pair, said change between the consecutive echocardiograms refers to a change in the cardiac function and/or structure of the heart as represented in the consecutive echocardiograms;
for each pair of consecutive echocardiograms, determining (306) an abstract representation of each echocardiogram by performing at layers of a convolutional deep learning network one or more convolutions and/or reductions on the echocardiograms in the pair, the abstract representation comprising one or more features indicative of the class of the pair; and
training (308) a predictive model to determine the class for a new pair of echocardiograms based on the abstract representations for the plurality of pairs of consecutive echocardiograms.

2. A computer-implemented method as claimed in claim 1, further comprising temporally aligning the pair of consecutive echocardiograms with respect to a cardiac cycle.

3. A computer-implemented method as claimed in claim 1 or 2, wherein each echocardiogram comprises a plurality of image frames, the method further comprising interpolating one or more image frames for one or both of the echocardiograms such that each echocardiogram comprises the same number of image frames.

4. A computer-implemented method as claimed in any of the preceding claims, further comprising:
receiving a new echocardiogram for a subject;
obtaining a previous echocardiogram for the subject;
using the predictive model to determine a class for the new echocardiogram and the previous echocardiogram.

5. A computer-implemented method as claimed in claim 4, further comprising controlling an interface to provide a notification to a user if the determined class of the new pair of echocardiograms indicates there is a change.

6. A computer-implemented method as claimed in claim 4 or claim 5, wherein the step of obtaining a previous echocardiogram comprises receiving an associated indication of the content of the previous echocardiogram, the method further comprising controlling an interface to provide the associated indication and/or the content to a user if the determined class of the new pair of echocardiograms indicates there is no change.

7. A computer-implemented method as claimed in any preceding claim, wherein each echocardiogram comprises a plurality of sequences, each sequence representing a different view of the subject, and wherein analysing each pair of consecutive echocardiograms further comprises joining the plurality of sequences of each echocardiogram to form a single image.

8. A computer-implemented method as claimed in any of claims 1 to 7, wherein each echocardiogram comprises a plurality of sequences, each sequence representing a different view of the subject, and wherein analysing each pair of consecutive echocardiograms further comprises comparing each of the plurality of sequences in one echocardiogram of the pair with each of the plurality of sequences in the other echocardiogram of the pair.

9. A computer-implemented method as claimed in any of the preceding claims, wherein each echocardiogram comprises a plurality of sequences, each sequence representing a different view of the subject, and wherein the method further comprises associating a view tag with each of the plurality of sequences, wherein the view tag is indicative of the view of the subject represented by the sequence.

10. A computer-implemented method as claimed in claim 9, wherein a view tag is one of: parasternal long axis, parasternal short axis, apical four chamber, apical five chamber, apical two chamber, apical three chamber, sub costal, and supra sternal notch.

11. A computer program product comprising a computer readable medium having computer readable code embodied therein, the computer readable code being configured such that, on execution by a suitable computer or processor, the computer or processor is caused to perform the method of any of claims 1 - 10.

12. An apparatus for analysing echocardiograms, the apparatus (100) comprising a processor (102) configured to:
obtain a plurality of pairs of consecutive echocardiograms for a plurality of subjects from a database (200), each echocardiogram having an associated indication, comprising one or more observational or diagnostic statements input by a user of the content of the echocardiogram;
analyse each pair of consecutive echocardiograms to determine an associated class by automatic comparison between said observational or diagnostic statements associated with each of the pair of consecutive echocardiograms, the class indicating whether there is a change or no change between the consecutive echocardiograms in the pair, said change between the consecutive echocardiograms refers to a change in the cardiac function and/or structure of the heart as represented in the consecutive echocardiograms;
for each pair of consecutive echocardiograms, determine an abstract representation of each echocardiogram by performing at layers of a convolutional deep learning network one or more convolutions and/or reductions on the echocardiograms in the pair, the abstract representation comprising one or more features indicative of the class of the pair; and
train a predictive model to determine the class for a new pair of echocardiograms based on the abstract representations for the plurality of pairs of consecutive echocardiograms.

## Patentansprüche

1. Computerimplementiertes Verfahren zur Analyse von Echokardiogrammen, wobei das Verfahren umfasst:
Erhalten (302) einer Vielzahl von Paaren aufeinanderfolgender Echokardiogramme für eine Vielzahl von Subjekten aus einer Datenbank (200), wobei jedes Echokardiogramm eine zugehörige Indikation aufweist, die eine oder mehrere Beobachtungs- oder Diagnoseaussagen umfasst, die von einem Benutzer des Inhalts des Echokardiogramms eingegeben werden;
Analysieren (304) jedes Paares aufeinanderfolgender Echokardiogramme, um durch automatischen Vergleich zwischen den Beobachtungs- oder Diagnoseaussagen, die jedem Paar aufeinanderfolgender Echokardiogramme zugeordnet sind, eine zugehörige Klasse zu bestimmen, wobei die Klasse angibt, ob eine Änderung oder keine Änderung zwischen den aufeinanderfolgenden Echokardiogrammen im Paar vorliegt, wobei sich die Änderung zwischen den aufeinanderfolgenden Echokardiogrammen auf eine Änderung der Herzfunktion und/oder Struktur des Herzens bezieht, wie in den aufeinanderfolgenden Echokardiogrammen dargestellt;
für jedes Paar aufeinanderfolgender Echokardiogramme, Bestimmen (306) einer abstrakten Darstellung jedes Echokardiogramms durch Durchführen einer oder mehrerer Faltungen und/oder Reduktionen auf den Echokardiogrammen in dem Paar auf Schichten eines Faltungs-Tiefenlernnetzwerks, wobei die abstrakte Darstellung ein oder mehrere Merkmale umfasst, die die Klasse des Paares angeben; und
Trainieren (308) eines Vorhersagemodells, um die Klasse für ein neues Paar von Echokardiogrammen basierend auf den abstrakten Darstellungen für die Vielzahl von Paaren aufeinanderfolgender Echokardiogramme zu bestimmen.

2. Computerimplementiertes Verfahren nach Anspruch 1, das außerdem das zeitliche Ausrichten des Paares aufeinanderfolgender Echokardiogramme in Bezug auf einen Herzzyklus umfasst.

3. Computerimplementiertes Verfahren nach Anspruch 1 oder 2, wobei jedes Echokardiogramm eine Vielzahl von Bildrahmen umfasst, wobei das Verfahren weiterhin das Interpolieren eines oder mehrerer Bildrahmen für eines oder beide der Echokardiogramme umfasst, sodass jedes Echokardiogramm die gleiche Anzahl von Bildrahmen umfasst.

4. Computerimplementiertes Verfahren nach einem der vorhergehenden Ansprüche, weiterhin umfassend:
Empfangen eines neuen Echokardiogramms für ein Subjekt;
Erhalten eines früheren Echokardiogramms für das Subjekt;
Verwenden des Vorhersagemodells, um eine Klasse für das neue Echokardiogramm und das vorherige Echokardiogramm zu bestimmen.

5. Computerimplementiertes Verfahren nach Anspruch 4, das außerdem die Steuerung einer Schnittstelle umfasst, um einem Benutzer eine Benachrichtigung bereitzustellen, wenn die ermittelte Klasse des neuen Paares von Echokardiogrammen anzeigt, dass eine Änderung vorliegt.

6. Computerimplementiertes Verfahren nach Anspruch 4 oder Anspruch 5, wobei der Schritt des Erhaltens eines früheren Echokardiogramms den Empfang einer zugehörigen Angabe des Inhalts des vorherigen Echokardiogramms umfasst, wobei das Verfahren weiterhin die Steuerung einer Schnittstelle umfasst, um die zugeordnete Angabe und/oder den Inhalt einem Benutzer bereitzustellen, wenn die ermittelte Klasse des neuen Paares von Echokardiogrammen anzeigt, dass keine Änderung vorliegt.

7. Computerimplementiertes Verfahren nach einem der vorhergehenden Ansprüche, wobei jedes Echokardiogramm eine Vielzahl von Sequenzen umfasst, wobei jede Sequenz eine andere Ansicht des Subjekts darstellt, und wobei das Analysieren jedes Paares aufeinanderfolgender Echokardiogramme außerdem das Zusammenfügen der Vielzahl von Sequenzen jedes Echokardiogramms umfasst, um ein einzelnes Bild zu bilden.

8. Computerimplementiertes Verfahren nach einem der Ansprüche 1 bis 7, wobei jedes Echokardiogramm eine Vielzahl von Sequenzen umfasst, wobei jede Sequenz eine andere Ansicht des Subjekts darstellt, und wobei das Analysieren jedes Paares aufeinanderfolgender Echokardiogramme außerdem den Vergleich jeder der Vielzahl von Sequenzen in einem Echokardiogramm des Paares mit jeder der Vielzahl von Sequenzen im anderen Echokardiogramm des Paares umfasst.

9. Computerimplementiertes Verfahren nach einem der vorhergehenden Ansprüche, wobei jedes Echokardiogramm eine Vielzahl von Sequenzen umfasst, wobei jede Sequenz eine andere Ansicht des Subjekts darstellt, und wobei das Verfahren weiterhin das Zuordnen eines Ansichts-Tags zu jeder der Vielzahl von Sequenzen umfasst, wobei das Ansichts-Tag die Ansicht des durch die Sequenz dargestellten Subjekts anzeigt.

10. Computerimplementiertes Verfahren nach Anspruch 9, wobei ein Ansichts-Tag eines von: parasternale lange Achse-, parasternale kurze Achse-, apikale Vierkammer-, apikale Fünfkammer-, apikale Zweikammer-, apikale Dreikammer-, subkostale- und suprasternale Kerbe-Ansichts-Tag ist.

11. Computerprogrammprodukt, das ein computerlesbares Medium mit darin enthaltenem computerlesbarem Code umfasst, wobei der computerlesbare Code so konfiguriert ist, dass der Computer oder Prozessor bei Ausführung durch einen geeigneten Computer oder Prozessor veranlasst wird, das Verfahren nach einem der Ansprüche 1 bis 10 auszuführen.

12. Vorrichtung zur Analyse von Echokardiogrammen, wobei die Vorrichtung (100) einen Prozessor (102) umfasst, der konfiguriert ist zum:
Erhalten einer Vielzahl von Paaren aufeinanderfolgender Echokardiogramme für eine Vielzahl von Subjekten aus einer Datenbank (200), wobei jedes Echokardiogramm eine zugehörige Indikation aufweist, die eine oder mehrere Beobachtungs- oder Diagnoseaussagen umfasst, die von einem Benutzer des Inhalts des Echokardiogramms eingegeben wurden;
Analysieren jedes Paares aufeinanderfolgender Echokardiogramme, um durch automatischen Vergleich zwischen den Beobachtungs- oder Diagnoseaussagen, die jedem Paar aufeinanderfolgender Echokardiogramme zugeordnet sind, eine zugehörige Klasse zu bestimmen, wobei die Klasse angibt, ob eine Änderung oder keine Änderung zwischen den aufeinanderfolgenden Echokardiogrammen in dem Paar vorliegt, wobei sich die Änderung zwischen den aufeinanderfolgenden Echokardiogrammen auf eine Änderung der Herzfunktion und/oder der Struktur des Herzens bezieht, wie sie in den aufeinanderfolgenden Echokardiogrammen dargestellt wird;
für jedes Paar aufeinanderfolgender Echokardiogramme, Bestimmen einer abstrakten Darstellung jedes Echokardiogramms, durch Durchführen einer oder mehrerer Faltungen und/oder Reduktionen auf den Echokardiogrammen im Paar auf Schichten eines Faltungs- Tiefenlernnetzwerks, wobei die abstrakte Darstellung ein oder mehrere Merkmale umfasst, die die Klasse des Paares angeben; und
Trainieren eines Vorhersagemodells, um die Klasse für ein neues Paar von Echokardiogrammen basierend auf den abstrakten Darstellungen für die Vielzahl von Paaren aufeinanderfolgender Echokardiogramme zu bestimmen.

## Revendications

1. Procédé mis en œuvre par ordinateur pour analyser des échocardiogrammes, le procédé consistant à:
obtenir (302) une pluralité de paires d'échocardiogrammes consécutifs pour une pluralité de sujets à partir d'une base de données (200), chaque échocardiogramme ayant une indication associée, comprenant une ou plusieurs déclarations d'observation ou de diagnostic entrées par un utilisateur du contenu de l'échocardiogramme;
analyser (304) chaque paire d'échocardiogrammes consécutifs pour déterminer une classe associée par comparaison automatique entre lesdites déclarations d'observation ou de diagnostic associées à chacune des paires d'échocardiogrammes consécutifs, la classe indiquant s'il y a un changement ou non entre les échocardiogrammes consécutifs dans le paire, ledit changement entre les échocardiogrammes consécutifs fait référence à un changement dans la fonction cardiaque et/ou la structure du coeur tel que représenté dans les échocardiogrammes consécutifs;
pour chaque paire d'échocardiogrammes consécutifs, déterminer (306) une représentation abstraite de chaque échocardiogramme en effectuant au niveau des couches d'un réseau d'apprentissage profond convolutif une ou plusieurs convolutions et/ou réductions sur les échocardiogrammes de la paire, la représentation abstraite comprenant une ou plusieurs caractéristiques indicatives de la classe de la paire; et
entraîner (308) un modèle prédictif pour déterminer la classe pour une nouvelle paire d'échocardiogrammes sur la base des représentations abstraites pour la pluralité de paires d'échocardiogrammes consécutifs.

2. Procédé mis en œuvre par ordinateur tel que revendiqué dans la revendication 1, comprenant en outre l'alignement temporel de la paire d'échocardiogrammes consécutifs par rapport à un cycle cardiaque.

3. Procédé mis en œuvre par ordinateur tel que revendiqué dans la revendication 1 ou 2, dans lequel chaque échocardiogramme comprend une pluralité de trames d'image, le procédé comprenant en outre l'interpolation d'une ou plusieurs trames d'image pour l'un des échocardiogrammes ou les deux échocardiogrammes de telle sorte que chaque échocardiogramme comprenne le même nombre de trames d'images.

4. Procédé mis en œuvre par ordinateur tel que revendiqué dans l'une quelconque des revendications précédentes, consistant en outre à:
recevoir un nouvel échocardiogramme pour un sujet;
obtenir un échocardiogramme précédent pour le sujet;
utiliser le modèle prédictif pour déterminer une classe pour le nouvel échocardiogramme et l'échocardiogramme précédent.

5. Procédé mis en œuvre par ordinateur tel que revendiqué dans la revendication 4, consistant en outre à commander une interface pour fournir une notification à un utilisateur si la classe déterminée de la nouvelle paire d'échocardiogrammes indique qu'il y a un changement.

6. Procédé mis en œuvre par ordinateur tel que revendiqué dans la revendication 4 ou la revendication 5, dans lequel l'étape d'obtention d'un échocardiogramme précédent comprend la réception d'une indication associée du contenu de l'échocardiogramme précédent, le procédé comprenant en outre la commande d'une interface pour fournir l'indication associée et/ou le contenu à un utilisateur si la classe déterminée de la nouvelle paire d'échocardiogrammes indique qu'il n'y a aucun changement.

7. Procédé mis en œuvre par ordinateur tel que revendiqué dans l'une quelconque des revendications précédentes, dans lequel chaque échocardiogramme comprend une pluralité de séquences, chaque séquence représentant une vue différente du sujet, et dans lequel l'analyse de chaque paire d'échocardiogrammes consécutifs comprend en outre l'assemblage de la pluralité de séquences de chaque échocardiogramme pour former une image unique.

8. Procédé mis en œuvre par ordinateur tel que revendiqué dans l'une quelconque des revendications 1 à 7, dans lequel chaque échocardiogramme comprend une pluralité de séquences, chaque séquence représentant une vue différente du sujet, et dans lequel l'analyse de chaque paire d'échocardiogrammes consécutifs comprend en outre la comparaison de chacune de la pluralité de séquences dans un échocardiogramme de la paire avec chacune de la pluralité de séquences dans l'autre échocardiogramme de la paire.

9. Procédé mis en œuvre par ordinateur tel que revendiqué dans l'une quelconque des revendications précédentes, dans lequel chaque échocardiogramme comprend une pluralité de séquences, chaque séquence représentant une vue différente du sujet, et dans lequel le procédé comprend en outre l'association d'une balise de vue à chacune de la pluralité de séquences, où la balise de vue est indicative de la vue du sujet représenté par la séquence.

10. Procédé mis en œuvre par ordinateur tel que revendiqué dans la revendication 9, dans lequel une balise de vue est l'une parmi: une balise de vue d'axe long parasternal, d'axe court parasternal, apicale des quatre chambres, apicale des cinq chambres, apicale des deux chambres, apicale des trois chambres, sous-costale, et d'échancrure suprasternale.

11. Produit de programme informatique comprenant un support lisible par ordinateur dans lequel est incorporé un code lisible par ordinateur, le code lisible par ordinateur étant configuré de telle sorte que, lors de son exécution par un ordinateur ou un processeur approprié, l'ordinateur ou le processeur est amené à exécuter le procédé selon l'une quelconque des revendications 1 à 10.

12. Appareil d'analyse d'échocardiogrammes, l'appareil (100) comprenant un processeur (102) configuré pour:
obtenir une pluralité de paires d'échocardiogrammes consécutifs pour une pluralité de sujets à partir d'une base de données (200), chaque échocardiogramme ayant une indication associée, comprenant une ou plusieurs déclarations d'observation ou de diagnostic entrées par un utilisateur du contenu de l'échocardiogramme;
analyser chaque paire d'échocardiogrammes consécutifs pour déterminer une classe associée par comparaison automatique entre lesdites déclarations d'observation ou de diagnostic associées à chacune des paires d'échocardiogrammes consécutifs, la classe indiquant s'il y a un changement ou non entre les échocardiogrammes consécutifs dans la paire, ledit changement entre les échocardiogrammes consécutifs fait référence à un changement dans la fonction cardiaque et/ou la structure du coeur tel que représenté dans les échocardiogrammes consécutifs;
pour chaque paire d'échocardiogrammes consécutifs, déterminer une représentation abstraite de chaque échocardiogramme en effectuant au niveau des couches d'un réseau d'apprentissage profond convolutif une ou plusieurs convolutions et/ou réductions sur les échocardiogrammes de la paire, la représentation abstraite comprenant une ou plusieurs caractéristiques indicatives de la classe de la paire; et
entraîner un modèle prédictif pour déterminer la classe pour une nouvelle paire d'échocardiogrammes sur la base des représentations abstraites pour la pluralité de paires d'échocardiogrammes consécutifs.
